# EUROPÄISCHE PATENTANMELDUNG

(11) **EP 3 851 034 A1**
(43) Veröffentlichungstag der Anmeldung: **21.07.2021**
(21) Anmeldenummer: 20152235.6
(22) Anmeldetag: 16.01.2020
(51) Int. Cl.: A61B 5/024, A61B 5/0245, A61B 5/04, A61B 5/00, A61N 1/04

(54) **VORRICHTUNG MIT WENIGSTENS EINER ELEKTRODENEINHEIT FÜR EINE ELEKTROSTIMULATION ODER EINE DATENERFASSUNG DIAGNOSTISCHER GERÄTE**

(71) Anmelder: Pierenkemper GmbH, 35630 Ehringshausen (DE)
(72) Erfinder: Peinze, Jonas, 9485 Nendeln (LI); Pierenkemper, Roger, 35630 Ehringshausen (DE)
(74) Vertreter: Knefel, Cordula

(57) **Zusammenfassung**

Die Erfindung betrifft eine Vorrichtung für eine Elektrostimulation oder für eine Datenerfassung diagnostischer Geräte mit wenigstens einer Elektrodeneinheit, bei der die Elektrodeneinheit folgende Schichten aufweist:
a) Grundschicht mit wenigstens einer in oder an der Grundschicht angeordneten Elektrode, und
b) Schicht mit einem der folgenden Schichtaufbauten:
b1)
- Kunststoffschicht und
- Schicht aus Gewirk und
- Kunststoffschicht
oder
b2)
- Schicht aus Gewirk und
- Kunststoffschicht
oder
b3)
- Kunststoffschicht und
- Schicht aus Gewirk
und dass die Schichten a) und b) fest miteinander verbunden sind.

## Beschreibung

Die Erfindung mit wenigstens einer Elektrodeneinheit betrifft eine Vorrichtung für eine Elektrostimulation oder für eine Datenerfassung diagnostischer Geräte.

Zum Stand der Technik (DE 10 2014 108 315 A1) gehören Behandlungsvorrichtungen und Behandlungsverfahren, beispielsweise für ein Gesicht. Bei diesen Behandlungen wird eine Elektrostimulation mittels in einer Gesichtsmaske in einem gestrickten Träger eingestrickten Elektroden durchgeführt. Die Gesichtsmaske wird hierbei aus einem Strickmaterial oder einem Gewirk, beispielsweise einem Kettengewirk hergestellt. Die Elektroden bestehen aus elektrisch leitfähigen Fäden, die unmittelbar bei der Herstellung der Gesichtsmaske mit eingestrickt werden. Diese zum Stand der Technik gehörende Vorrichtung weist den Nachteil auf, dass die Gesichtsmaske vor der Herstellung vollständig konzipiert sein muss, da die Elektrode in die Gesichtsmaske eingestrickt wird. Ein nachträgliches Anpassen an verschiedene Träger in Bezug auf die Lage der Elektroden ist nicht möglich.

### Weiterhin gehört zum Stand der Technik

(DE 20 2012 102 393 U1) eine Vorrichtung zur komplexen Elektromyostimulation. Diese zum Stand der Technik gehörende Vorrichtung umfasst einen Anzug, eine Stimulationseinheit, eine Bedieneinheit sowie eine die Bedieneinheit und die Stimulationseinheit zur Signalübertragung verbindende Kommunikationseinheit. Auch dieser Anzug besteht aus einem Kettengewirk. Bei diesem Anzug ist die Elektrodeneinheit an dem Anzug angeordnet. Die Elektroden bestehen aus metallisierten Polymerfasern. Die Kontaktelektroden können auch aus leitfähigen Polymermassen bestehen. Diese zum Stand der Technik gehörenden Kontaktelektroden weisen den Nachteil auf, dass die Polymermassen leicht brüchig werden, insbesondere bei intensiver Nutzung des Anzuges. Werden die Polymermassen brüchig, sind die Elektroden nicht mehr funktionsfähig und der Anzug ist für die Elektrostimulation nicht mehr zu gebrauchen. Zudem entsprechen die derzeit am Markt erhältlichen Materialien nicht den hohen Zytotoxizitätsanforderungen, die an Produkte dieser Art gestellt werden.

Das der Erfindung zugrundeliegende technische Problem besteht darin, eine Vorrichtung für eine Elektrostimulation oder für eine Datenerfassung diagnostischer Geräte mit wenigstens einer Elektrodeneinheit anzugeben, welche eine gute Leitfähigkeit des Elektrodenmaterials gewährleistet bei einer großen Haltbarkeit der Elektroden.

Dieses technische Problem wird durch eine Vorrichtung mit den Merkmalen gemäß Anspruch 1 gelöst.

Die erfindungsgemäße Vorrichtung mit wenigstens einer Elektrodeneinheit für eine Elektrostimulation oder für eine Datenerfassung diagnostischer Geräte zeichnet sich dadurch aus, dass die Elektrodeneinheit folgende Schichten aufweist:
a) Grundschicht mit wenigstens einer in oder an der Grundschicht angeordneten Elektrode, und
b) Schicht mit einem der folgenden Schichtaufbauten:
   b1)
      - Kunststoffschicht und
      - Schicht aus Gewirk und
      - Kunststoffschicht
      oder
   b2)
      - Schicht aus Gewirk und
      - Kunststoffschicht
      oder
   b3)
      - Kunststoffschicht und
      - Schicht aus Gewirk
und dass die Schichten a) und b) fest miteinander verbunden sind.

Die erfindungsgemäße Vorrichtung mit wenigstens einer Elektrodeneinheit kann zum einen für eine Elektrostimulation verwendet werden. Die wenigstens eine Elektrode der Elektrodeneinheit wird mit Strom beaufschlagt und kann aus diesem Grunde für eine Elektrostimulation verwendet werden. Es ist jedoch auch möglich, eine Datenerfassung mit der erfindungsgemäßen Vorrichtung mit der wenigstens einer Elektrodeneinheit durchzuführen, beispielsweise eine Datenerfassung mittels EKG (Elektrokardiogramm), EMG (Elektromyographie) oder zum Beispiel zur Messung von Bioimpedanzen usw..

Die erfindungsgemäße Vorrichtung weist folgenden Aufbau mit folgenden Schichten auf:
a) Grundschicht mit wenigstens einer in oder an der Grundschicht angeordneten Elektrode und
b) Schicht mit einem der folgenden Schichtaufbauten:
   b1)
      - Kunststoffschicht und
      - Schicht aus Gewirk und
      - Kunststoffschicht
      oder
   b2)
      - Schicht aus Gewirk und
      - Kunststoffschicht
      oder
   b3)
      - Kunststoffschicht und
      - Schicht aus Gewirk

Bei der erfindungsgemäßen Elektrodeneinheit sind die Schichten a) und b) fest miteinander verbunden. Das bedeutet, dass die Schicht a) mit der Schicht b1) oder die Schicht a) mit der Schicht b2) oder die Schicht a) mit der Schicht b3) fest verbunden ist. Vorteilhaft sind die Schichten der Schicht b1) oder der Schicht b2) oder der Schicht b3) ebenfalls fest miteinander verbunden.

Durch den erfindungsgemäßen Aufbau ist gewährleistet, dass die Schicht b) eine Schicht aus einem Gewirk aufweist. Hierdurch bleibt dieser Schichtaufbau stabil, auch wenn die Grundschicht mit der weiteren Schicht häufig in ihrer Grundform verändert, beispielsweise gebogen, geknickt, gestreckt, gestaucht oder dergleichen wird.

Die erfindungsgemäße Vorrichtung kann beispielsweise an einem Anzug für eine Elektrostimulation angeordnet sein. Dieser Anzug enthält vorteilhaft wenigstens eine Elektrodeneinheit. Die wenigstens eine Elektrodeneinheit wird je nach Anordnung an dem Anzug mehr oder weniger stark tordiert oder gestreckt oder gestaucht oder in anderer Weise beansprucht. Durch Ausgestaltung der Elektrodeneinheit mit der Schicht aus Gewirk mit wenigstens einer Kunststoffschicht bleibt die Elektrodeneinheit auch über einen langen Benutzungszeitraum stabil und funktionsfähig, auch bei einer starken mechanischen Beanspruchung.

Gemäß einer vorteilhaften Ausführungsform der Erfindung wird die Elektrode in die Grundschicht eingestickt. Das Einsticken hat den Vorteil, dass die Elektrode in der gewünschten Form in der Grundschicht angeordnet werden kann. Diese gestickte Elektrode ist sehr haltbar.

Gemäß einer vorteilhaften Ausführungsform wird die Elektrode mit elektrisch leitfähigen Fäden in die Grundschicht eingestickt. Vorteilhaft werden die elektrisch leitfähigen Fäden lediglich in einer Oberfläche der Grundschicht eingestickt. Sie werden dabei nicht durch die gesamte Grundschicht durchgestickt. Auf der gegenüberliegenden Seite können nicht elektrisch leitfähige Haltefäden angeordnet sein. Grundsätzlich besteht jedoch auch die Möglichkeit, dass die elektrisch leitfähigen Fäden durch die gesamte Grundschicht durchgehend angeordnet sind.

Gemäß weiteren vorteilhaften Ausführungsformen kann die wenigstens eine Elektrode in der Grundschicht auch durch Drucken, Nähen, Kleben und/oder Einlegen und Fixieren in oder an der Grundschicht angeordnet sein.

Die Elektrode wird vorteilhaft lediglich auf einer Seite der Grundschicht angeordnet. Das heißt, dass die Grundschicht auf einer Seite die elektrisch leitfähige Elektrode aufweist. Auf der anderen Seite ist die Grundschicht nicht oder nur in einem geringen Maße elektrisch leitfähig.

Gemäß einer besonders bevorzugten Ausführungsform der Erfindung ist vorgesehen, dass die wenigstens eine Elektrode in oder an der vorgefertigten Grundschicht angeordnet ist. Diese Ausführungsform weist den Vorteil auf, dass die Elektrode auf der vorgefertigten Grundschicht der Elektrodeneinheit in beliebiger Form aufgestickt werden kann. Die Grundschicht an sich ist fertig hergestellt. Anschließend wird die wenigstens eine Elektrode in oder an dieser vorgefertigten Grundschicht angeordnet. Ist zum Beispiel eine Anpassung von Elektroden an bestimmte Körperregionen erforderlich, kann dieses in dem Stickvorgang festgelegt werden.

Es ist nicht von vornherein notwendig, das Design der gesamten Elektrodeneinheit schon bei der Herstellung der Grundschicht festzulegen. Erst bei der Herstellung der Elektrodeneinheit wird die endgültige Form in der Grundschicht festgelegt. Auf diese Art und Weise können Elektrodeneinheiten mit unterschiedlichen Designs preiswert hergestellt werden.

Die wenigstens eine Elektrode wird vorteilhaft aus wenigstens einem elektrisch leitfähigen Faden gebildet. Es können mehrere Elektroden mit einem Faden oder Fadenstrang hergestellt sein. Diese Elektroden sind dann in Reihe geschaltet. Es besteht auch die Möglichkeit, eine oder mehrere Elektroden aus jeweils einem elektrisch leitfähigen Faden herzustellen. In diesem Fall können eine oder mehrere Elektroden parallel geschaltet werden. Die Elektroden können einzeln oder zusammen oder gruppenweise ansteuerbar ausgebildet werden.

Gemäß einer weiteren vorteilhaften Ausführungsform der Erfindung ist vorgesehen, dass die wenigstens eine Kunststoffschicht elektrisch leitfähig ausgebildet ist. Hierdurch ist es möglich, eine Stromübertragung über die Kunststoffschicht vorzunehmen.

Gemäß einer weiteren vorteilhaften Ausführungsform der Erfindung ist vorgesehen, dass die wenigstens eine Kunststoffschicht aus einem thermoplastischen Material und/oder aus thermoplastischen Elastomeren und/oder aus leitfähigen Silikonen gebildet ist.

Diese Materialien sind besonders flexibel und können mit dem Anzug oder einer Manschette oder dergleichen verformt werden. Darüber hinaus bleibt die Flexibilität über einen sehr langen Zeitraum erhalten.

Gemäß einer weiteren vorteilhaften Ausführungsform der Erfindung ist vorgesehen, dass die wenigstens eine Kunststoffschicht elektrisch leitfähige Partikel aufweist. Hierdurch ist die wenigstens eine Kunststoffschicht ebenfalls elektrisch leitfähig. Durch die Kunststoffschicht kommt der Nutzer der erfindungsgemäßen Vorrichtung, beispielsweise der Patient oder ein Sportler, schneller ins Schwitzen. Durch die vom Schweiß gebildete Feuchtigkeit wird die Leitfähigkeit erhöht.

Es können sämtliche in den Schichten b1), b2) oder b3) angeordneten Kunststoffschichten elektrisch leitfähige Partikel aufweisen. Es besteht jedoch auch die Möglichkeit, dass nur ein Teil der in den Schichten b1), b2) oder b3) angeordneten Kunststoffschichten elektrisch leitfähige Partikel aufweisen.

Die elektrisch leitfähigen Partikel sind vorteilhaft homogen oder wenigstens annähernd homogen in der wenigstens einen Kunststoffschicht angeordnet.

Gemäß einer weiteren vorteilhaften Ausführungsform der Erfindung ist vorgesehen, dass die elektrisch leitfähigen Partikel aus Silber und/oder Graphit bestehen. Diese Partikel lassen sich einfach in die Kunststoffschicht einbringen und erhöhen die elektrische Leitfähigkeit der wenigstens einen Kunststoffschicht.

Eine weitere vorteilhafte Ausführungsform der Erfindung sieht vor, dass die Grundschicht aus einem Vlies besteht. Es besteht jedoch auch die Möglichkeit, andere Materialien für die Grundschicht zu verwenden. Da die Elektrodeneinheit beispielsweise durch ein Heißpressverfahren hergestellt wird, sollte die Grundschicht hitzebeständig sein. Sie sollte sich bei einem Thermopressverfahren nicht zu sehr zusammendrücken.

Eine weitere vorteilhafte Ausführungsform der Vorrichtung sieht vor, dass die wenigstens eine Kunststoffschicht als Polyurethanfolie ausgebildet ist. Polyurethanfolie lässt sich besonders preiswert herstellen. Darüber hinaus können in der Polyurethanfolie die elektrisch leitfähigen Partikel gut und einfach angeordnet werden.

Eine besonders bevorzugte Ausführungsform der Erfindung sieht vor, dass die wenigstens eine Schicht aus Gewirk
- Gaze und/oder
- elastische Gaze und/oder
- gitterförmiges Material und/oder
- gitterförmiges elastisches Material
aufweist und/oder
- aus Gaze und/oder
- aus elastischer Gaze und/oder
- einem gitterförmigen Material und/oder
- einem gitterförmigen elastischen Material
gebildet ist.

Das bedeutet, dass in den Schichten b1), b2) oder b3) vorteilhaft eine Schicht aus Gaze und/oder aus elastischer Gaze und/oder einem gitterförmigen Material und/oder einem gitterförmigen elastischen Material gebildet ist.

Die Schichten b1), b2) oder b3) können auch Gaze und/oder gitterförmiges Material, vorzugsweise elastische Gaze und/oder gitterförmiges elastisches Material aufweisen.

Die Gaze und/oder das gitterförmige Material sind vorzugsweise elastisch.

Das Gewirk, beispielsweise die Gaze und/oder das gitterförmige Material, vorzugsweise elastische Material, gewährleisten, dass die wenigstens eine Kunststoffschicht nicht brüchig wird oder höheren mechanischen Belastungen standhält. Diese Schicht aus Gewirk bewirkt eine hohe Stabilität der Elektrodeneinheit bei gleichzeitiger Flexibilität der Elektrodeneinheit, so dass sich die Elektrodeneinheit an Körperteile des Patienten oder Sportlers anpassen kann. Das Gewirk, beispielsweise die Gaze und/oder das gitterförmige Material sind vorteilhaft in einer Richtung, besonders vorteilhaft jedoch in zwei Richtungen, beispielsweise in x- und in y-Richtung elastisch ausgebildet, um eine bestmögliche Anpassung der Elektrodeneinheit an ein Körperteil oder an den Körper eines Patienten oder Sportlers zu gewährleisten.

Vorteilhaft ist das Gewirk, beispielsweise die Gaze oder das gitterförmige Material aus Polyester gebildet. Dieses Material ist preiswert, lang haltbar und lässt sich gut als Gewirk, beispielsweise in Form von Gaze oder gitterförmigem Material mit der entsprechenden Elastizität ausbilden.

Vorteilhaft zeichnet sich die erfindungsgemäße Vorrichtung dadurch aus, dass die Schichten als
- durch ein Thermopressverfahren miteinander verschweißte Schichten oder
- durch ein Thermopressverfahren mit einem Haftvermittler fest verbundene Schichten oder
- durch Aufkaschieren fest miteinander verbundene Schichten oder
- durch ein Hochfrequenzschweißen oder ein Ultraschallschweißen fest miteinander verbundene Schichten ausgebildet sind.

Die verschiedenen Schichten mittels der vorgenannten Verfahren fest miteinander zu verbinden, weist den Vorteil auf, dass das Verfahren preiswert durchführbar ist und die Flexibilität der Elektrodeneinheit trotzdem gewährleistet ist. Darüber hinaus werden feste, unlösbare Verbindungen zwischen den Schichten gebildet.

Gemäß einer weiteren vorteilhaften Ausführungsform der Erfindung ist vorgesehen, dass die Vorrichtung mit der Grundschicht an einem Ganzkörperanzug oder Teilkörperanzug angeordnet ist. Der Ganzkörperanzug deckt den Mittelkörper wie auch die Extremitäten ab, während ein Teilkörperanzug beispielsweise in Form eines T-Shirts, eines Pullovers oder einer Art Hose ausgebildet sein kann. Durch diese Ausbildung ist es möglich, beispielsweise für einen Sportler einen Ganzkörperanzug mit der erfindungsgemäßen Vorrichtung zu versehen, so dass während des Sportes beispielsweise eine Elektrostimulation stattfinden kann.

Gemäß einer anderen vorteilhaften Ausführungsform ist vorgesehen, dass die Vorrichtung mit der Grundschicht auf einer Manschette angeordnet ist. Diese Manschette kann beispielsweise an den Extremitäten eines Sportlers oder eines Patienten angeordnet werden, um ebenfalls bei einer sportlichen Aktivität eine Elektrostimulation durchzuführen oder eine Elektrostimulation für therapeutische Zwecke durchzuführen oder Daten beispielsweise in Form eines EKGs oder EMGs zu erfassen.

Gemäß einer weiteren vorteilhaften Ausführungsform der Erfindung ist vorgesehen, dass der Ganzkörperanzug oder die Manschette aus
- Polyester und Elasthan oder
- Polyamid 6 oder Elasthan oder
- Polyamid 6.6 und Elasthan oder
- Polyurethan und Elasthan
besteht.

Diese Materialien sind besonders vorteilhaft, da der Ganz- oder Teilkörperanzug oder die Manschette hierdurch flexibel ausgestaltet sind, um Scherbewegungen, Torsionsbewegungen, Streck- und/oder Stauchbewegungen des Ganz- oder Teilkörperanzuges oder der Manschette bei Bewegung des Sportlers oder Patienten mitzumachen, so dass die Elektrodeneinheit mit der wenigstens einen Elektrode ständig in Körperkontakt bleibt.

Gemäß einer weiteren vorteilhaften Ausführungsform der Erfindung weist die Elektrode einen Widerstand bezüglich der elektrischen Leitfähigkeit von weniger als 100 Ohm pro 10 Zentimeter Länge der Elektrode auf. Dies ist vorteilhaft, da die Elektroden hierdurch eine große Leitfähigkeit aufweisen, so dass eine wirksame Elektrostimulation stattfinden kann.

Eine besonders bevorzugte Ausführungsform der erfindungsgemäße Vorrichtung mit der wenigstens einen Elektrodeneinheit sieht vor, dass eine gestickte Elektrode verwendet wird, die in einem Heizpressverfahren mit den weiteren Schichten verbunden wird.

Wird lediglich eine Kunststofffolie verwendet, die leitende Partikel enthält, entstehen durch die ständige Bewegung der flächigen Elektrode leicht Beschädigungen. Aus diesem Grunde ist es vorteilhaft, ein Gewirk, beispielsweise in Form einer Gaze oder eines gitterförmigen elastischen Materials zusätzlich vorzusehen.

Die Grundschicht mit der Elektrode ist ebenfalls vorteilhaft niederohmig ausgebildet, das bedeutet sie weist vorteilhaft eine hohe Leitfähigkeit und einen sehr geringen Widerstand auf.

Das Gewirk, beispielsweise die Gaze, vorzugsweise elastisch, oder das gitterförmige, vorzugsweise elastische Material besteht vorteilhaft aus Polyester oder anderen Materialien. Die Gaze ist ebenfalls vorteilhaft elastisch ausgebildet.

Die wenigstens eine Kunststoffschicht besteht vorteilhaft aus einer PU-Folie (Polyurethan), die besonders vorteilhaft elektrisch leitfähige Partikel, beispielsweise Graphit und Silber enthält. Die PU-Folie kann auch aus thermoplastischem Material oder aus thermoplastischen Elastomeren wie auch aus leitfähigen Silikonen bestehen.

Trägt der Sportler einen Ganzkörper- oder Teilkörperanzug, kommt er bei der sportlichen Tätigkeit ins Schwitzen. Hierdurch wird die Grundschicht, die in Richtung der Haut angeordnet ist, mit Schweiß durchsetzt. Durch die Erhöhung der Feuchtigkeit wird auch die Leitfähigkeit verbessert.

Die Kunststoffschichten werden vorteilhaft auf das Gewirk, beispielsweise die Gaze und/oder das gitterförmige elastische Material aufkaschiert. Es besteht auch die Möglichkeit, die Elektrodeneinheit im Thermoverfahren auf die Kunststofffolie aufzukaschieren.

Wird die erfindungsgemäße Vorrichtung in einem Ganzkörper- oder Teilkörperanzug angeordnet, weist dieser Anzug vorteilhaft außen eine nicht leitfähige Schicht, beispielsweise eine Schicht aus Polyurethan auf. Die erfindungsgemäße Vorrichtung mit der Elektrodeneinheit kann eine Verbindung zum Anzug beispielsweise mittels Verkleben oder auch mittels eines Thermoverfahrens aufweisen. Besonders vorteilhaft sind die Kunststofffolien aus einem dünnen flexiblen Material gebildet, welches eine hohe Leitfähigkeit aufweist, beispielsweise mit einem Widerstand von weniger als 100 Ohm pro 10 cm Länge der Elektrode.

Die elektrisch leitfähigen Fasern, die beispielsweise eingestickt, eingelegt oder eingedruckt werden, können beispielsweise aus Edelstahl oder Kupfer bestehen.

Weitere Merkmale und Vorteile der Erfindung ergeben sich anhand der zugehörigen Zeichnungen, in der mehrere Ausführungsbeispiele der Vorrichtung dargestellt sind, ohne die Erfindung auf diese Ausführungsbeispiele zu beschränken. In den Zeichnungen zeigen:
- Fig. 1: einen Teilkörperanzug mit Elektroden-einheiten;
- Fig. 2: eine Elektrodeneinheit im Querschnitt;
- Fig. 3: ein geändertes Beispiel einer Elektrodeneinheit im Querschnitt;
- Fig. 4: ein geändertes Beispiel einer Elektrodeneinheit im Querschnitt.

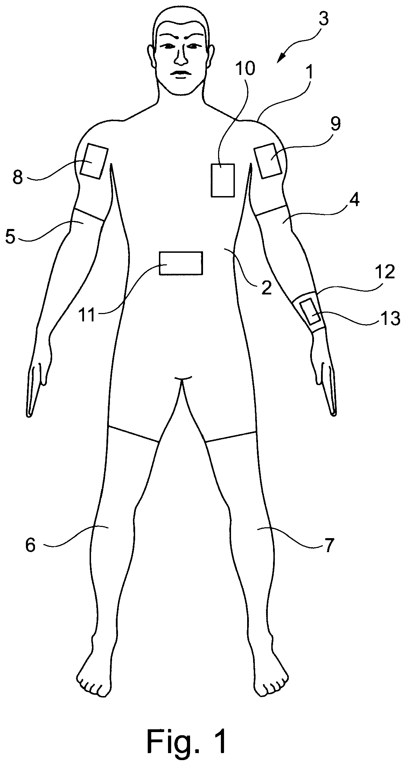

Fig. 1 zeigt einen Teilkörperanzug 1, der einen Rumpf 2 einer Person 3 abdeckt. Extremitäten 4 bis 7 bleiben teilweise frei von dem Teilkörperanzug 1. An dem Teilkörperanzug 1 sind Elektrodeneinheiten 8, 9, 10, 11 angeordnet. Die Elektrodeneinheiten 8, 9 sind im Oberarmbereich angeordnet, die Elektrode 11 im Bauchbereich und die Elektrode 10 im Herzbereich der Person 3. Darüber hinaus trägt die Person 3 an der Extremität 4, das heißt einem Arm, eine Manschette 12, an der eine Elektrodeneinheit 13 angeordnet ist. Die Elektrodeneinheiten 8, 9 und 11 können für eine Elektrostimulation verwendet werden. Die Elektrodeneinheiten 10, 13 können beispielsweise für eine Datenerfassung verwendet werden, beispielsweise für ein EKG.

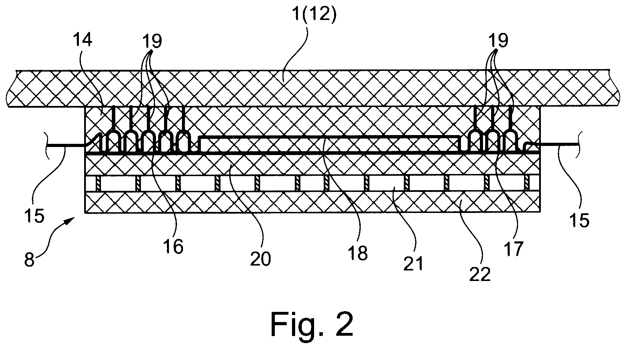

Fig. 2 zeigt die Elektrodeneinheit 8, die an dem Anzug 1 oder der Manschette 12 angeordnet ist. Die Elektrodeneinheit 1 besteht aus einer Grundschicht 14. In der Grundschicht 14 sind aus einem elektrisch leitfähigen Faden 15 zwei Elektroden 16, 17 eingestickt. Der Faden 15 tritt aus beiden Seiten der Grundschicht 14 hervor, damit ein elektrischer Stromkreis gebildet werden kann. Zwischen den Elektroden 16, 17 ist ebenfalls eine elektrisch leitfähige Verbindung 18 mit dem Faden 15 hergestellt. Der Faden 15 ist lediglich auf der dem Anzug abgewandten Seite der Grundschicht 14 eingestickt. Der Faden 15 wird mit Haltefäden 19, die nicht elektrisch leitfähig sind, in der Grundschicht 14 fixiert. Grundsätzlich besteht auch die Möglichkeit, dass die Elektroden 16, 17 aus zwei Fäden 15 gebildet werden, so dass zwei Stromkreise vorhanden sind. Die Elektroden 16, 17 sind in diesem Fall einzeln ansteuerbar.

Die Grundschicht 14 besteht aus Vlies und ist fest an dem Anzug 1 oder der Manschette 12 mittels Kleben, Thermopressverfahren oder dergleichen befestigt. Auf der dem Anzug 1 oder der Manschette 12 abgewandten Seite der Grundschicht ist eine Kunststoffschicht 20 angeordnet. An der Grundschicht 20 ist eine weitere Schicht aus einem Gewirk, im vorliegenden Fall aus einer Gaze 21 angeordnet. Darüber hinaus ist eine weitere Kunststoffschicht 22 vorgesehen.

Die Gaze 21 besteht vorteilhaft aus einem elastischen Material. Sie kann aus Polyester oder anderen Materialien bestehen.

Die Kunststofffolien 20, 22 sind vorteilhaft als Polyurethanfolie ausgebildet. In den Kunststoffschichten 20, 21 können entweder in der einen Schicht oder in der anderen Schicht oder in beiden Schichten elektrisch leitfähige Partikel wie zum Beispiel Graphit oder Silber (nicht dargestellt) angeordnet sein. Die Kunststoffschichten 20, 22 können auch aus thermoplastischem Material oder aus thermoplastischen Elastomeren wie auch aus leitfähigen Silikonen bestehen.

Die Schichten 14, 20, 21, 22 können in einem Heizpressverfahren miteinander fest verbunden werden. Sämtliche beschriebenen Schichten 1 (12, 14, 20, 21, 22) sind fest miteinander verbunden.

Die Fig. 2 zeigt den Aufbau der Schichten wie im Anspruch 1 mit dem Schichtaufbau b1) beschrieben.

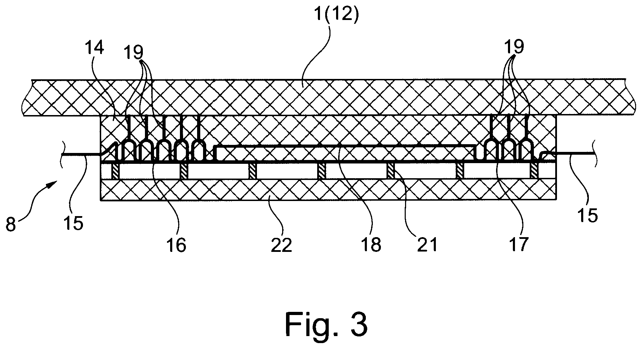

In Fig. 3 sind gleiche Teile wie in Fig. 2 mit den gleichen Bezugszeichen versehen. Die Elektrodeneinheit 8 besteht aus der Grundschicht 14 mit den eingestickten Elektroden 16, 17. Auf der Grundschicht 14 ist auf der dem Anzug 1 beziehungsweise der Manschette 12 abgewandten Seite die Gaze 21 angeordnet. Weiterhin ist eine Kunststoffschicht 22 vorgesehen. Dieser Aufbau entspricht dem Aufbau b2) des Anspruches 1. Die Materialien sind die Materialien, die zur Fig. 2 beschrieben wurden.

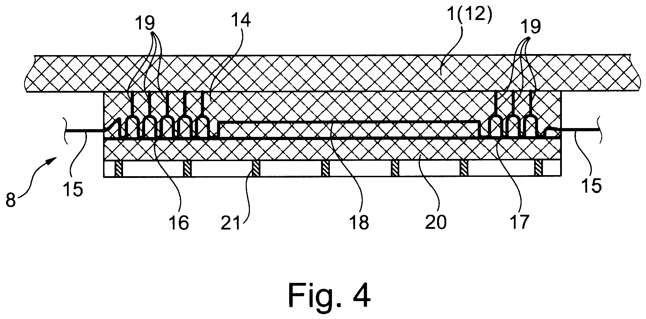

Fig. 4 zeigt einen weiteren möglichen Aufbau. Gleiche Teile wie in den Fig. 2 und 3 sind mit gleichen Bezugszeichen versehen. An dem Anzug 1 beziehungsweise der Manschette 12 ist die Grundschicht 14 mit den Elektroden 16, 17 angeordnet. Auf der dem Anzug 1 oder der Manschette 12 abgewandten Seite der Grundschicht 14 ist die Kunststoffschicht 20 angeordnet. Auf der Kunststoffschicht 20 ist die Gaze 21 angeordnet. Dieser Aufbau entspricht dem Aufbau b3) des Anspruches 1.

Die Grundschicht 14 kann aus einem Vlies, einem Gewirk, einem Gestrick oder dergleichen bestehen. Die Elektroden 16, 17 sind vorteilhaft eingestickt in die Grundschicht 14. Sie können jedoch auch gedruckt, eingelegt, festgeklebt oder dergleichen sein.

Durch die Anordnung des Schichtaufbaus auf der Grundschicht auf der Seite, die dem Anzug oder der Manschette abgewandt ist mit wenigstens einer Kunststoffschicht 20, 22 und der Gaze 21, wird eine flexible Elektrodeneinheit 8 gebildet, die auch durch langen Gebrauch mit wiederkehrenden Scher- und/oder Torsionskräften und/oder Streckungen und/oder Stauchungen lange haltbar ist.

### Bezugszahlen

- 1: Teilkörperanzug
- 2: Rumpf
- 3: Person
- 4: Extremität (Arm)
- 5: Extremität (Arm)
- 6: Extremität (Bein)
- 7: Extremität (Bein)
- 8: Elektrodeneinheit
- 9: Elektrodeneinheit
- 10: Elektrodeneinheit
- 11: Elektrodeneinheit
- 12: Manschette
- 13: Elektrodeneinheit
- 14: Grundschicht
- 15: leitfähiger Faden
- 16: Elektrode
- 17: Elektrode
- 18: elektrisch leitfähige Verbindung
- 19: Haltefäden
- 20: Kunststoffschicht
- 21: Gaze
- 22: Kunststoffschicht

## Patentansprüche

1. Vorrichtung für eine Elektrostimulation oder für eine Datenerfassung diagnostischer Geräte mit wenigstens einer Elektrodeneinheit,
**dadurch gekennzeichnet, dass** die Elektrodeneinheit (8, 9, 10, 11, 13) folgende Schichten aufweist:
a) Grundschicht (14) mit wenigstens einer in oder an der Grundschicht angeordneten Elektrode (16, 17), und
b) Schicht mit einem der folgenden Schichtaufbauten:
b1)
- Kunststoffschicht (20) und
- Schicht (21) aus Gewirk und
- Kunststoffschicht (22)
oder
b2)
- Schicht aus Gewirk (21) und
- Kunststoffschicht (22)
oder
b3)
- Kunststoffschicht (20) und
- Schicht (21) aus Gewirk
und dass die Schichten a) und b) (14, 20, 21, 22)fest miteinander verbunden sind.

2. Vorrichtung nach Anspruch 1, **dadurch gekennzeichnet, dass** die Elektrode (16, 17) durch Sticken, Drucken, Nähen, Kleben und/oder Einlegen und Fixieren in oder an der Grundschicht (14) angeordnet ist.

3. Vorrichtung nach Anspruch 1 oder 2, **dadurch gekennzeichnet, dass** die Elektrode (16, 17) in oder an der vorgefertigten Grundschicht (14) angeordnet ist.

4. Vorrichtung nach einem der vorhergehenden Ansprüche, **dadurch gekennzeichnet, dass** die wenigstens eine Kunststoffschicht (20, 22) elektrisch leitfähig ausgebildet sind.

5. Vorrichtung nach einem der vorhergehenden Ansprüche, **dadurch gekennzeichnet, dass** die wenigstens eine Kunststoffschicht (20, 22) aus einem thermoplastischen Material und/oder aus thermoplastischen Elastomeren und/oder aus leitfähigen Silikonen gebildet ist.

6. Vorrichtung nach einem der vorhergehenden Ansprüche, **dadurch gekennzeichnet, dass** die wenigstens eine Kunststoffschicht (20, 22) elektrisch leitfähige Partikel aufweist.

7. Vorrichtung nach Anspruch 6, **dadurch gekennzeichnet, dass** die elektrisch leitfähigen Partikel aus Silber und/oder Graphit bestehen.

8. Vorrichtung nach einem der vorhergehenden Ansprüche, **dadurch gekennzeichnet, dass** die Grundschicht (14) aus einem Vlies besteht.

9. Vorrichtung nach einem der vorhergehenden Ansprüche, **dadurch gekennzeichnet, dass** die wenigstens eine Kunststoffschicht (20, 22) als Polyurethan-Folie ausgebildet ist.

10. Vorrichtung nach Anspruch 1, **dadurch gekennzeichnet, dass** die Schicht aus Gewirk aus Polyester gebildet ist.

11. Vorrichtung nach einem der vorhergehenden Ansprüche, **dadurch gekennzeichnet, dass** die wenigstens eine Schicht (21) aus Gewirk
- Gaze und/oder
- elastische Gaze und/oder
- gitterförmiges Material und/oder
- gitterförmiges elastisches Material
aufweist oder aus
- Gaze und/oder
- elastischer Gaze und/oder
- gitterförmigen Material und/oder
- gitterförmigen elastischem Material gebildet ist.

12. Vorrichtung nach einem der vorhergehenden Ansprüche, **dadurch gekennzeichnet, dass** die Schichten (14, 20, 21, 22) als
- durch ein Thermopressverfahren miteinander verschweißte Schichten (14, 20, 21, 22) oder
- durch ein Thermopressverfahren mit einem Haftvermittler fest verbundene Schichten (14, 20, 21, 22) oder
- durch Aufkaschieren fest miteinander verbundene Schichten (14, 20, 21, 22) oder
- durch ein Hochfrequenzschweißen oder ein Ultraschallschweißen fest miteinander verbundene Schichten (14, 20, 21, 22)
ausgebildet sind.

13. Vorrichtung nach einem der vorhergehenden Ansprüche, **dadurch gekennzeichnet, dass** die Vorrichtung mit der Grundschicht (14) an einem Ganzkörperanzug oder Teilkörperanzug (1) oder an einer Manschette (12) angeordnet ist.

14. Vorrichtung nach einem der Ansprüche 13, **dadurch gekennzeichnet, dass** der Ganzkörperanzug oder der Teilkörperanzug (1) oder die Manschette (12) aus
- Polyester und Elasthan oder
- Polyamid 6 und Elasthan oder
- Polyamid 6.6 und Elasthan oder
- Polyurethan und Elasthan
besteht.
